(19) Europäisches Patentamt
European Patent Office
Office européen des brevets

(11) Publication number: 0 149 327
A2

(12) # EUROPEAN PATENT APPLICATION

(21) Application number: 84308412.0

(22) Date of filing: 04.12.84

(51) Int. Cl.⁴: C 07 D 209/52
A 61 K 31/557
//C07C177/00

(30) Priority: 16.01.84 US 571238

(43) Date of publication of application:
24.07.85 Bulletin 85/30

(84) Designated Contracting States:
BE CH DE FR GB IT LI NL SE

(71) Applicant: THE UPJOHN COMPANY
301 Henrietta Street
Kalamazoo, Michigan 49001(US)

(72) Inventor: Anderson, Bradley Dale
c/o The Upjohn Company 301 Henrietta Street
Kalamazoo Michigan 49001(US)

(74) Representative: Perry, Robert Edward et al,
GILL JENNINGS & EVERY 53-64 Chancery Lane
London WC2A 1HN(GB)

(54) **A prostaglandin salt and compositions containing it.**

(57) The potassium salt of 6,9-deepoxy-6,9-(phenylimino)-$\Delta^{6,8}$-prostaglandin $I_1$. This salt can be formulated into pharmaceutical compositions, e.g. in the form of an aerosol. The novel salt has a number of advantageous physical properties.

EP 0 149 327 A2

This invention relates to a prostaglandin salt and to compositions containing it.

The solid-state chemical stability of a compound sometimes can be influenced by the selection of an appropriate salt form. However, no established general principles exist. A review of information relating to the selection of "Pharmaceutical Salts" is given by Berge et al, J. Pharm. Sci. 66 (1977) 1-19.

US-A-4294759 discloses the free acid of 6,9-deepoxy-6,9-(phenylimino)-$\Delta^{6,8}$-prostaglandin $I_1$ and analogues, and also their utility as anti-asthmatic agents.

The novel compound of the present invention is the potassium salt of 6,9-deepoxy-6,9-(phenylimino)-$\Delta^{6,8}$-prostaglandin $I_1$. Its formula is given below.

A pharmaceutical composition according to the invention comprises the potassium salt of the invention and a pharmaceutical carrier. Such a composition is preferably in the form of a powdered or liquid aerosol.

The compound and compositions of this invention are useful in the prophylactic or therapeutic treatment of allergy of a reagin or non-reagin mediated nature, e.g. in treating asthma, allergic rhinitis, food allergy and urticaria. In general, the compound and compositions of the invention may be used, formulated and administered in the same manner as the pharmaceutically-useful compounds described in US-A-4294759 (of which the disclosure and, in particular, the section from column 12 line 45 to column 14 line 53, is herein incorporated by reference). The novel compound can also prevent or inhibit hypersecretion of mucus in the respiratory tract of warm-blooded animals.

By comparison with the free acid, the novel compound crystallises easily, and has a high melting point, high water-solubility, and low solubility in fluorinated and fluorochlorinated liquified propellants. The novel potassium salt has unexpectedly improved solid-state stability, e.g. during shelf-life or storage, as in aerosol suspensions, over the free acid or the sodium salt; relative data are tabulated below.

TABLE I

Physico-Chemical Data

| | Property | Value Desired | Free Acid | Sodium Salt | Potassium Salt |
|---|---|---|---|---|---|
| 1) | Ease of synthesis | Easily crystallized | -- | Acceptable | Acceptable |
| 2) | Characterizability | Passes profile (+ low moisture content after air equilibration) | Pass | (.82% $H_2O$)[a] | Pass (.42% $H_2O$) |
| 3) | Water solubility | Dissolves readily in water | 1 µg/ml | Soluble | Soluble |
| 4) | Trichlorotrifluoroethane solubility | Low | .38 µg/ml | -- | .043 µg/ml |
| 5) | Melting point (micronizability) | High (>100°C) | 135-140 | >175 | >175 |
| 6) | Solid state stability (see Table II) | <2% degradation (2 months at 33°C) | >10% | >>50% | <2% |
| 7) | Specific gravity | 1.3-1.6[b] | -- | 1.18 | 1.20 |

a) Initial analyses acceptable but later assays (HPLC) unacceptable due to solid-state decomposition.

b) Specific gravities below this range may require incorporation of other high density solids into an aerosol suspension.

TABLE II

Solid-State Stability

| Storage Conditions | Time (days) | Percent of Theory: Free Acid | Sodium Salt | Potassium Salt | Potassium Salt Micronized |
|---|---|---|---|---|---|
| Dark, 4° | 0 | -- | 101 | 100 | -- |
| | 19 | -- | 87 | -- | -- |
| | 46 | -- | 77 | -- | -- |
| | 66 | -- | -- | 102 | -- |
| | 189 | -- | -- | 101 | -- |
| Dark, 33° | 0 | 100 | 101 | 100 | 100 |
| | 19 | 98 | 61 | 100 | -- |
| | 40 | 94 | -- | 100 | -- |
| | 66 | 89 | -- | 100 | -- |
| | 100 | -- | -- | 97 | 97 |
| | 189 | 65 | -- | 98 | -- |
| | 216 | -- | -- | 98 | 98 |
| | 289 | 46 | -- | 96 | -- |
| | 405 | 19 | -- | 96 | -- |
| High intensity light, 33° | 0 | 100 | 101 | 100 | -- |
| | 19 | 55 | 3 | 48 | -- |
| Dark, 75% R.H.[a], 30° (visual observation) | 0 | Free flowing crystalline solids | | | |
| | 19 | same | same | glass | |
| Trichloro-trifluoro-ethane suspension, dark, 30° | 0 | 100 | -- | 100 | -- |
| | 26 | 94 | -- | 100 | -- |

a) R.H. means relative humidity.

As seen from the tabulated data the most striking and dramatic difference among the three compounds is their solid-state stability. The rank order of stability of the compounds when protected from light during storage, which is the recommended manner of storage, at 33° was potassium salt > free acid > sodium salt.

The solubility of the compounds in trichlorotrifluoroethane, a high molecular weight fluorochlorinated alkane liquid propellant, was determined as follows. Suspensions of each compound were prepared at concentrations of ~1 mg/ml in trichlorotrifluoroethane in 30 ml glass vials sealed using polytetrafluoroethylene lined caps. These vials were wrapped with aluminum foil and shaken at ~25°C. After 26 days shaking the samples were filtered. Three successive 5 ml fractions were collected from the filter and analyzed to test for adsorption. (No adsorption was observed.)

The 5 ml samples were transferred to 5 ml volumetric flasks and solvent was evaporated under nitrogen. The residue was redissolved in 1 ml methanol and diluted to 5 ml with 30% $CH_3CN$. Samples were assayed by high pressure liquid chromatography.

The solid-state stability studies were performed as follows. Approximately 10-15 mg of the free acid or a salt was placed in either clear glass vials or amber vials wrapped in aluminum foil and stored under various conditions as listed below:

a) Refrigerated at about 4°C (dark).

b) High Intensity Light Box at about 33°C and relative humidity <15%. (Light Box contained 6-15 watt fluorescent bulbs 11.42 cm above the powder surface.) Sample vials were placed on their sides with powder spread evenly on the vial wall.

c) 30°C/75% relative humidity/dark - vials were capped loosely.

d) Suspended in trichlorotrifluoromethane and shaken continuously for 26 days.

At various times, 1-1.5 mg samples were dissolved in methanol, diluted to a prescribed volume with water, and analyzed by high pressure liquid chromatography versus reference standard of the free acid stored at -20°C. Chromatographic conditions were similar to those described previously.

To obtain specific gravity data approximate densities were obtained using a volume displacement procedure. To a 5 ml weighed pycnometer was added 200-300 mg of salt and acetonitrile added to

fill. The salt suspensions were sonicated to remove entrapped air and equilibrated to 20°C in a water bath. The salt densities were estimated by standard methods of calculation.

Example 1 6,9-Deepoxy-6,9-(phenylimino)-$\Delta^{6,8}$-prostaglandin $I_1$, potassium salt

A tetrahydrofuran-$H_2O$ solution (10 ml:10ml) of 6,9-deepoxy-6,9-(phenylimino)-$\Delta^{6,8}$-prostaglandin $I_1$ free acid was adjusted to pH 7.5 with concentrated KOH. An additional 90 ml of water was added, pH was readjusted to 8.5, and the solution was freeze dried. The freeze-dried product was recrystallized in methanol-acetonitrile, dried at 55°C/high vac overnight, and equilibrated with laboratory atmosphere. M.P. 176° dec.

Example 2 6,9-Deepoxy-6,9-(phenylimino)-$\Delta^{6,8}$-prostaglandin $I_1$, sodium salt

A solution of 6,9-deepoxy-6,9-(phenylimino)-$\Delta^{6,8}$-prostaglandin $I_1$ free acid in 5.4 ml of 1N methanolic NaOH was filtered, washing the filter with 10 ml of methanol. Acetonitrile was slowly added to the solution while stirring resulting in crystallization of an off-white solid. After 2-3 hours stirring the solid was collected, dried overnight at 55°C, then equilibrated with laboratory atmosphere for about 4 hours. M.P. 177°C.

Example 3

The following illustrates an aerosol suspension formulation for inhalation of the compound of this invention.

| | | Weight Percent |
|---|---|---|
| (a) | 6,9-deepoxy-6,9-(phenylimino)-$\Delta^{6,8}$-prostaglandin $I_1$, potassium salt | 0.714 |
| (b) | sorbitan trioleate | 0.500 |
| (c) | dichlorotetrafluoroethane | 98.786 |
| | | 100.000 |

Materials (a) to (c) are packaged in suitable (aluminum) containers equipped with a metering valve designed to meter 50 mcl per dose, an equivalent of 0.5 mg of compound (a).

Example 4

To prepare a typical powder for inhalation, 2 g of 6,9-deepoxy-6,9-(phenylimino)-$\Delta^{6,8}$-prostaglandin $I_1$, potassium salt and sufficient lactose to make 8 g of mixture are comminuted and placed in hard

filled capsules designed for use in a device that permits inspiration of the powder by a patient. Each capsule will deliver 40 mg of powder which is equivalent to 10 mg of drug.

FORMULA

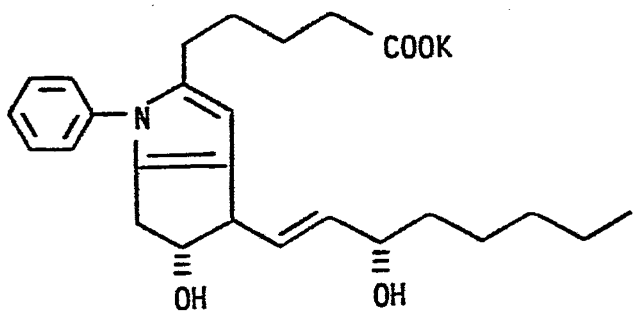

CLAIMS

1. The potassium salt of 6,9-deepoxy-6,9-(phenylimino)-$\Delta^{6,8}$-prostaglandin $I_1$.

2. A pharmaceutical composition which comprises the potassium salt of 6,9-deepoxy-6,9-(phenylimino)-$\Delta^{6,8}$-prostaglandin $I_1$ and a pharmaceutical carrier.

3. A composition according to claim 2, in the form of a powdered aerosol.

4. A composition according to claim 2, in the form of a liquid aerosol.

5. A composition which comprises the potassium salt of 6,9-deepoxy-6,9-(phenylimino)-$\Delta^{6,8}$-prostaglandin $I_1$ and a liquid propellant.

6. A suspension of the potassium salt of 6,9-deepoxy-6,9-(phenylimino)-$\Delta^{6,8}$-prostaglandin $I_1$ in a liquid propellant.

7. A composition which comprises the potassium salt of 6,9-deepoxy-6,9-(phenylimino)-$\Delta^{6,8}$-prostaglandin $I_1$ and a fluorinated or fluorochlorinated alkane liquid propellant.

8. A suspension of the potassium salt of 6,9-deepoxy-6,9-(phenylimino)-$\Delta^{6,8}$-prostaglandin $I_1$ in a fluorinated or fluorochlorinated alkane liquid propellant.